(19) Europäisches Patentamt
European Patent Office
Office européen des brevets

(11) **EP 2 818 562 A1**

(12) **EUROPEAN PATENT APPLICATION**

(43) Date of publication:
**31.12.2014 Bulletin 2015/01**

(51) Int Cl.:
***C12R 1/38*** *(2006.01)*      ***C12N 9/10*** *(2006.01)*
***C12P 7/62*** *(2006.01)*

(21) Application number: **13173575.5**

(22) Date of filing: **25.06.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Dritte Patentportfolio Beteiligungsgesellschaft mbH & Co. KG**
**12529 Schönefeld/OT Waltersdorf (DE)**

(72) Inventors:
• **Bassas Galià, Monica**
**1950 Sion (CH)**
• **Arias Rivas, Sagrario**
**2316 NZ Leiden (NL)**
• **Molinari, Gabriella**
**38304 Wolfenbüttel (DE)**
• **Timmis, Kenneth Nigel**
**38124 Braunschweig (DE)**

(74) Representative: **Held, Stephan**
**Meissner, Bolte & Partner GbR**
**Widenmayerstraße 47**
**80538 München (DE)**

(54) **Utilization of the novel, environmental isolate Pseudomonas sp. IPB-A36 for the efficient production of mcl/lcl-PHAs and specialty-PHAs**

(57)     The present application is directed at a microorganism of the genus *Pseudomonas* as deposited under DSM26198 with the Leibnitz Institute DSMZ. The present application is further directed at a process for the production of medium- and long-chain PHAs, comprising cultivating said microorganism in a culture medium comprising a carbon source and isolating the PHA from the microorganism. It has been observed that the microorganism allows for PHA production in high yield. In addition, the inventive microorganism possesses the valuable capability to efficiently incorporate unsaturated and/or aromatically modified fatty acids into the resulting PHAs. Accordingly, the inventive microorganism enables the production of chemically diverse PHAs, opening new fields of applications for these materials.

EP 2 818 562 A1

## Description

The present invention is in the field of biosynthesis of polyhydroxyalkanoates (PHA). The invention relates to a wild type microorganism of the genus *Pseudomonas* as deposited under DSM 26198 with the Leibnitz Institute DSMZ German collection of microorganisms and cell cultures. This microorganism has been proven to be of great utility in processes for the production of PHA. The microorganism is non-genetically modified and has been observed to be even capable to incorporate carbon sources comprising unsaturated and aromatic moieties to provide new PHA varieties with tuneable properties. The present invention is also directed to the use of this microorganism in a process for the production of medium- or long-chain PHA as well as to PHAs obtainable by such processes.

## Background of the invention

PHAs are polymers that are biodegradable and biocompatible thermoplastic materials (polyesters of 3-hydroxy fatty acids) produced from renewable resources with a broad range of industrial and biomedical applications (Williams & Peoples, 1996, Chemtech. 26: 38-44). PHAs are synthesized by a broad range of bacteria and have been extensively studied due to their potential use to substitute conventional petrochemical-based plastics to protect the environment from harmful effects of plastic wastes.

PHA can be divided into two groups according to the length of their side chains and their biosynthetic pathways. Those with short side chains, such as PHB, a homopolymer of (R)-3-hydroxybutyric acid units, are crystalline thermoplastics, whereas PHAs with long side chains are more elastomeric. The former have been known for about ninety years (Lemoigne & Roukhelman, 1925, Ann. Des Fermentation, 527-536), whereas the latter materials were discovered relatively recently (desmet et al., 1983, J. Bacteriol. 154: 870-878). Before this designation, however, PHA of microbial origin containing both (R)-3-hydroxybutyric acid units and longer side chain (R)-3-hydroxyacid units from 5 to 16 carbon atoms had been identified (Wallen & Rohweder, 1974, Environ. Sci. Technol. 8: 576-579). A number of bacteria, which produce copolymers of (R)-3-hydroxybutyric acid and one or more long side chain hydroxyl acid units containing from 5 to 16 carbon atoms, have been identified (Steinbüchel & Wiese, 1992, Appl. Microbiol. Biotechnol. 37: 691-697; Valentin et al., 1992, Appl. Microbiol. Biotechnol. 36: 507-514; Valentin et al., Appl. Microbiol. Biotechnol. 1994, 40: 710-716 ; Abe et al., 1994, Int. J. Biol. Macromol. 16: 115-119; Lee et al., 1995, Appl. Microbiol. Biotechnol. 42: 901-909; Kato et al., 1996, Appl. Microbiol. Biotechnol. 45: 363-370; Valentin et al., 1996, Appl. Microbiol. Biotechnol. 46: 261-267; US Patent No. 4,876,331). These copolymers can be referred to as PHB-co-HX (wherein X is a 3-hydroxyalkanoate or alkanoate or alkenoate of 6 or more carbons). A useful example of specific two-component copolymers is PHB-co-3-hydroxyhexanoate (PHB-co-3HH) (Brandl et al., 1989, Int. J. Biol. Macromol. 11: 49-55; Amos & McInerey, 1991, Arch. Microbiol. 155: 103-106; US Patent No. 5,292,860).

Although PHAs have been extensively studied because of their potential use as renewable resource for biodegradable thermoplastics and biopolymers (as mentioned above) and have been commercially developed and marketed (Hrabak, 1992, FEMS Microbiol. Rev. 103: 251-256), their production costs are much higher than those of conventional petrochemical-based plastics, which represents a major obstacle to their wider use (Choi & Lee, 1997, Bioprocess Eng. 17: 335-342). As described above, many bacteria produce PHAs, e.g. *Alcaligenes eutrophus, Alcaligenes latus, Azotobacter vinlandii, Pseudomonas acitophila, Pseudomonas oleovarans, Eschericha coli, Rhodococcus eutropha, Chromobacterlum violaceum, Chromatium vinosum, Alcanivorax borkumensis* etc. All PHA-producing bacteria known in the art produce intracellular PHA and accumulate it in PHA granules (SteinbUchel, 1991, Biomaterials, pp. 123-213). The main aspects, which render PHA production expensive and therefore unfavorable as compared to petrochemical-based plastic, are that it is difficult to produce the material in high yield and to recover the produced PHA from within the bacterial cells where it is accumulated. In order to reduce the total production costs of PHA, the development of an efficient recovery process was considered to be necessary, generally aiming at cell disruption (Lee, 1996, Biotech. Bioeng. 49: 1-14) by i) an appropriate solvent, ii) hypochlorite extraction of PHA and/or iii) digestion of non-PHA cellular materials.

At an industrial scale, the available microorganisms still provide relatively little PHA, which renders the production of PHA with these microorganisms economically non-feasible. All methods known in the art require large amounts of water during the production and in addition chemical reagents and/or enzymes for their recovery, which is an obstacle to reducing the production costs. Therefore, alternative strategies for PHA production are in urgent need.

In the recent past, strategies for the genetic modification of PHA-producing microorganisms have been developed, e.g. to enable the microorganisms to produce higher amounts of PHA. EP 1 913 135 A1 describes microorganisms, which have been genetically modified for example by knocking-out genes, which act on intermediates for the PHA production in a competitive manner to PHA synthases. By depleting the microorganism of enzymes, which interfere with PHA synthase for intermediates, it was possible to channel the intermediate conversion towards PHA.

Another approach was to introduce PHA synthases into microorganisms such as e.g. *Escherichia coli,* which in their wild type form are not capable to produce PHA (cf. Qi et al., 2007, FEMS Microbiol. Lett. 157: 155-162). In this case, a maximum PHA accumulation of about 15% CDW (cell dry weight) was observed in an *E. coli* LS1298 strain, when decanoate was used as the carbon source.

In a yet alternative approach, the PHA production was increased by knock-outs of PHA depolymerase genes, which in the microorganism *P. putida* KT2440 led to yields of about 4 g/L CDW with PHA accounting for up to 80% of the CDW (Cai et al., 2009, Bioresource Techn. 100: 2265-2270).

Despite of these advancements, the amount of PHA produced in these microorganisms compared with the resources necessary for their production is still relatively low. In addition, in some countries there are public reservations against genetically engineered microorganisms in general, which leads to problems in terms of acceptance of these materials. In particular for these countries, it would be advantageous to have wild type, i.e. non-genetically modified microorganisms, which produce PHA in high yields.

Most microorganisms, which have until now been described for PHA production, only accept saturated fatty acids as carbon sources for the production of PHAs. PHAs produced from regular substrates such as straight chain fatty acids with a chain length of 6 to about 20 carbon atoms usually exhibit glass transition temperatures of the polymers in the range of -30°C to -50°C. This limits their utility to applications, which are compatible with such glass transition temperatures. If the scope of substrates accepted by corresponding microorganisms for incorporation into PHA could be extended, this would have a great impact on the diversity of the properties of PHAs accessible from such microorganisms. In particular, if microorganism were available, which can also incorporate carbon sources resulting in modified properties of the PHA, this would have a great impact on the scope of applications for which the material could be used as a possible replacement for conventional petrochemical-based plastics.

The present application addresses these needs.

## Brief description of the invention

One aim of the present application is to provide a non-genetically modified (i.e. wild type) microorganism of the genus *Pseudomonas* deposited under DSM26198 with the Leibnitz Institute DSMZ, Inhoffenstr. 7B, 38124 Braunschweig, Germany. The microorganism *Pseudomonas sp.* IPB-A36 was isolated from an enrichment culture obtained from different contaminated (with hydrocarbons, Diesel and petroleum) soil samples from Canada and Australia within petroleum T138 (1%) as a substrate. This microorganism has been unexpectedly observed to allow for high-yield production of PHA and moreover to be capable to incorporate unconventional substrates, comprising e.g. aromatic and/or unsaturated moieties into PHA.

Under optimized conditions, the microorganism provided a biomass of more than 22 g/L CDW (cell dry weight) with a PHA content of 43 wt.-% corresponding to PHA total yields of more than 9 g/L.

The present application is further directed to a process for the production of medium and/or long chain PHA comprising:

- cultivating a microorganism of the genus *Pseudomonas* as deposited under DSM26198 with DSMZ in a culture medium comprising a carbon source and

- isolating the PHA from the microorganism.

Further aspects of the present application are directed at PHA obtainable from said process, wherein the PHA preferably comprises unsaturated and/or aromatic moieties and the use of the above-mentioned microorganism in a process for the production of medium- or long-chain PHA.

## Brief description of the drawings:

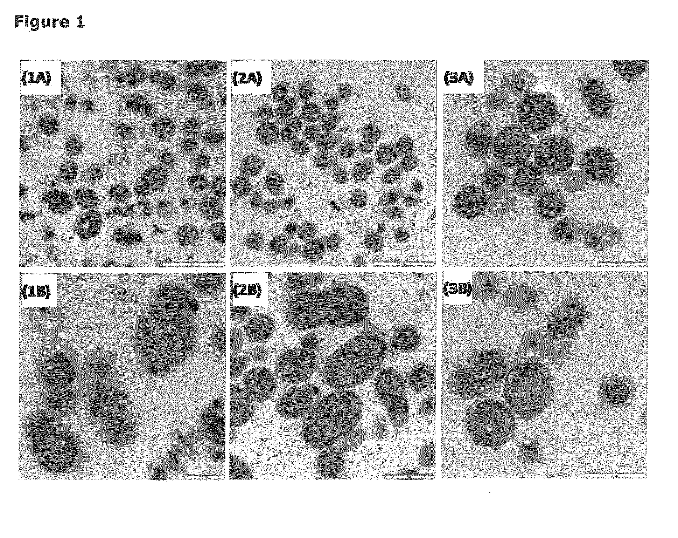

**Figure 1:** Transmission Electronic Microscopy (TEM)-image of strain *Pseudomonas sp.* IPB-A36 cultured in C-Y medium under different feeding conditions: (1A-B), 27 mM C11:1; (2A-B), 27+27 mM C11:1; (3A-B), 54 mM C11:1. After 72h of incubation at 30°C and 200 rpm, cells were collected for PHA extraction and 1 ml samples were prepared for TEM, respectively.

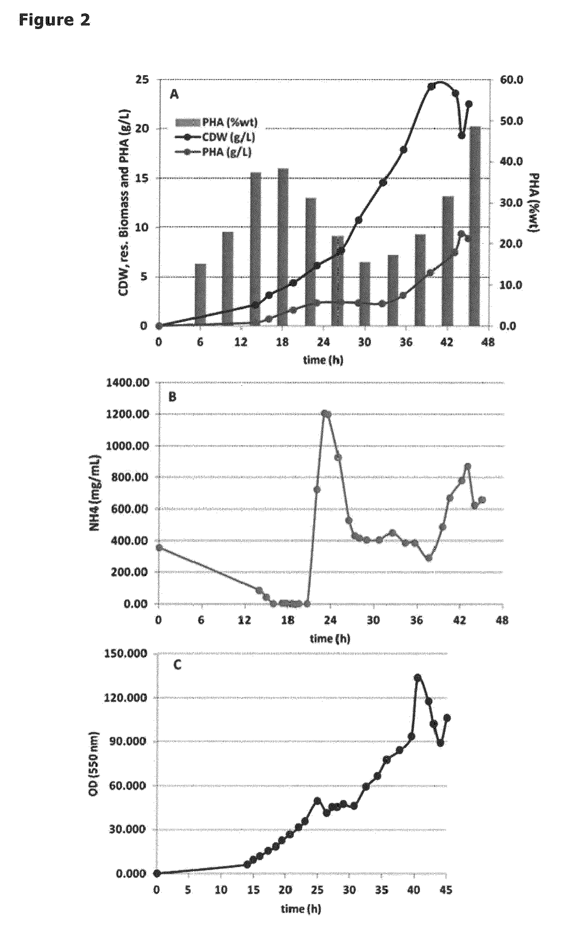

**Figure 2:** Fed-batch fermentation of strain Pseudomonas sp. IPB-A36 using 10-undecenoate as substrate. Kinetic of biomass and PHA production (A), ammonium consumption (B), and $OD_{550nm}$ measurements (C). Values are means of duplicates.

**Description of the invention**

Medium-chain, as this term is used in the context of the present invention, is intended to mean hydroxyl acid units ((R)-3-hydroxyacid units) with 5 to 13 carbon atoms. The term "long-chain PHA" is intended to encompass PHA, containing at least 14 carbon atoms per monomer.

In the course of the inventor's investigations, it had been discovered that the medium used for the fermentation of the inventive microorganism has a significant impact on the PHA productivity of the microorganism. From several production media tested, MM medium modified with 0.1% yeast extract (as described in Martinez-Blanko et al., 1990, J. Biol. Chem. 265: 7084-7090) provided the lowest PHA productivity when 10-undecenoate was used as the carbon source. Under the same conditions R2A medium as described by Reasoner & Geldreich (1985, Appl. Environ. Microbiol. 49: 1-7) provided significantly higher yields of PHA, while C-Y medium described in Choi et al. (1994, Appl. Environ. Microbiol. 60: 1245-1254) provided the highest yields in terms of PHA production. The yield of PHA from this medium exceeded the yield obtained with MM medium by a factor of more than 4. In the practice of the present invention, it is therefore preferred that the culture medium is C-Y medium as described by Choi et al..

In order to further improve the biomass and PHA yields, the content of nitrogen (N) and carbon (C) in medium was modified. Two different concentrations of nitrogen source and carbon source were assayed, considering the conditions provided by the preferred C-Y medium (5 mM ammonium sulphate and 27 mM 10-undecenoate) as standard conditions. It was observed that by increasing two-fold the concentrations of the nitrogen and carbon source, and maintaining the molar C/N ratio at 30, the PHA production could be further increased by a factor of more than 2. Accordingly, in yet another preferred embodiment of the present application, a modified C-Y medium with increased concentrations of both carbon and nitrogen source, is being used.

The inventive process is not subject to any relevant restrictions as concerns the carbon source to be employed for the production of PHA. Carbon sources, which are regularly employed for the production of PHA, can be used with the microorganism of the present application in the inventive process such as glycerol, sugars, pyruvate, and conventional fatty acids such as in particular fatty acids comprising 4 to 20 carbon atoms and preferably 8 to 18 fatty carbon atoms. It has been discovered, however, that the best yields of PHA in mg/L were obtained, if fatty acids or mixtures thereof are used as the carbon source. Consequently, a preferred process of the present application involves a carbon source, which comprises at least one C4 to C20 fatty acid, preferably a C8 to C18 fatty acid. The preferred saturated fatty acids to be used in the present application are butyric acid, valeric acid, hexanoic acid, heptanoic acid, caprylic acid, nonanoic acid, decanoic acid, lauric acid, myristic acid, palmitic acid, heptadecanoic acid, stearic acid, and aracidic acid.

It has further been discovered, that the inventive microorganism also accepts unsaturated fatty acids such as oleic acid and 10-undecenoic acid as a substrate. A preferred embodiment of the inventive process thus involves fatty acids as carbon sources, which comprise one or more unsaturated moieties, preferably a single unsaturated moiety. Representative unsaturated fatty acids comprise myristoleic acid, palmitoleic acid, sapienic acid, oleic acid, elaidic acid, vaccenic acid, linoleic acid, linoelaidic acid, a-linoleic acid, arachidonic acid, eicosapentaenoic acid, and undecenoic acid.

The inventive microorganism *Pseudomonas sp,* IPB-A36 also allows for the incorporation of carboxylic acids into the PHAs, which comprise an aromatic moiety. In a preferred process according to the present invention, the carbon source may thus comprise at least one carboxylic acid, comprising an aromatic moiety. This "carboxylic acid" may be used either in combination with of afore-mentioned fatty acids or as the sole substrate.

The carboxylic acid comprising an aromatic moiety is preferably a fatty acid, more preferably an ω-aryl substituted fatty acid, and most preferably an ω-phenyl substituted fatty acid. Said fatty acid preferably comprises 4 to 10 carbon atoms in the fatty acid chain. Preferred fatty acids of this type thus include e.g. 4-phenylbutyric acid, 5-phenylvaleric acid, 6-phenylhexanoic acid, 7-phenylheptanoic acid, 8-phenyloctanoic acid, 9-phenyloctanoic acid and 10-phenyldecanoic acid. Surprisingly, it has been observed that in the concentrations used for the fermentation, the carboxylic acids were non-toxic to the microorganism.

If a mixture of carboxylic acids comprising an aromatic moiety and fatty acids is used, it is further preferred that the carboxylic acid comprising an aromatic moiety is used in admixture with at least one C4 to C14 fatty acid and accounts for about 5 to 45% of the mixture. It had been observed, that if the concentration of aromatic carboxylic acid comprising an aromatic moiety is higher than the indicated range the yield of PHA in terms of PHA production in g/L and wt.-% is significantly lower than for a corresponding mixture wherein the carboxylic acid comprising an aromatic moiety accounts for about 5 to 45 wt.-% of the carbon source mixture. It had further been observed, that if the carboxylic acid comprising an aromatic moiety is in the indicated range, the yield of PHA both in terms of total PHA production and content with regard to the cell dry weight is comparable to fermentations wherein no carboxylic acid comprising an aromatic moiety is used.

In addition to the afore-mentioned carboxylic acids, it is also possible to include branched carboxylic acids into the PHAs such as for example tuberculostearic acid or 7-methyl-7-hexadecanoic acid.

In a preferred embodiment, these branched carboxylic acids are branched preferably at a carbon atom which is separated by at least 4 carbon atoms from the carboxylic acid moiety, preferably by at least 5 carbon atoms from the carboxylic acid moiety, while the carbon atoms closer to the carboxylic acid are unsubstituted.

The carbon source in the culture medium may comprise either only one of the above-mentioned carbon sources or a mixture of two ore more of these carboxylic acids. Preferably a mixture of at least one saturated and/or unsaturated fatty acid and at least one carboxylic acid comprising one or more unsaturated moieties is used. In this case, it is possible to add the respective carbon source in separate portions or as a mixture. An advantage of using more than one carbon source in the fermentation, in particular, mixtures of saturated, unsaturated and aromatic moiety comprising carboxylic acid, is that it is possible to precisely fine-tune the properties of the resulting PHA.

Further it is possible to add a mixture of carbon sources only at the beginning of the fermentation, in several individual lumps during the fermentation, or by continuously co-feeding the mixture. The latter alternative has the advantage that the carbon sources are incorporated into the PHA without substantial composition drift (i.e. the PHA formed at the beginning of the fermentation has the same composition as the PHA formed towards the end of the fermentation). Co-feeding the carbon source is thus preferred in the process of the present application.

If the process of the present application is employed in the context of a shake-flask or batch-process, it is further preferred, that the carbon to nitrogen (C/N) ratio in a culture medium is in the range of about 20 to 45, preferably in the range of about 25 to 35. If the (C/N) ratio is less than 20 or in excess of 45, the PHA yields of the resulting product were lower than in the preferred range.

In one embodiment of the present application, the carbon source is added as in a single lump to the cultivation mixture at the start of the cultivation. It was observed in this regard, that if the carbon source was added in e.g. two portions, one of which being added at the beginning of the cultivation and the second of which at a later stage, the PHA yield both in g/L and wt.-% was usually lower compared to a process wherein the carbon source was added as a single lump.

In the context of a shake-flask or batch-process it is further preferred, that the amount of carbon source added to the cultivating mixture is such that a concentration of the carbon source in the cultivating mixture in a range of about 20 to 60 mM, in particular in the range of about 45 to 55 mM, is obtained. If the carbon source is added to provide a concentration of less than 20 mM, the yield of PHA was lower than in fermentations wherein the concentration of the carbon source was in the indicated ranges. If the carbon source concentration is in excess of 60 mM, the environment becomes increasingly toxic to the cells, which negatively impacts their growth.

A further important parameter of the inventive process is the nitrogen content in the culture medium, as nitrogen is an important nutrient for the microorganisms, and PHA production is usually favoured under conditions, featuring an excess of carbon and a certain deficiency of e.g. nitrogen. In a preferred process of the present invention, an ammonium salt is used as the nitrogen source such as for example ammonium sulphate or ammonium hydroxide.

In a preferred process of the present invention, the ammonium concentration ($NH_4^+$) in the cultivation medium was in the range of about 8 to 30 mM, in particular in the range of about 10 to 20 mM. However, ultimately it is the C/N ratio, rather than the actual concentration of the nitrogen source, which has the largest impact on the strain's growth and PHA production.

A further important aspect of the present application is the oxygen concentration in the fermentation as the microorganisms consume oxygen to convert the carboxylic acids to 3-hydroxycarboxylic acids. In the practice of the present application, it is preferred that the partial pressure of oxygen ($pO_2$) is maintained between about 25% and 45%, preferably at about 30% in the cultivation medium, wherein % is mol-% and calculated based on the total gas dissolved in the cultivation medium.

With regard to the cultivation time, the present application is not subject to any relevant restrictions. The skilled practitioner will be aware, however, that during the cultivation, the amount of PHA produced at some stage will reach a maximum after which either the PHA-content declines or no longer changes. The skilled practitioner will be readily capable to determine the time wherein the amount of PHA accumulation in the microorganisms is highest. As a rule of a thumb, the maximum PHA accumulation in batch processes was usually reached after about 40 hours and before about 100 hours. Therefore, the cultivation is preferably carried out for a time of not less than 48 h and not more than 96 h, preferably for not less than 60 h to not more than 84 h and most preferably about 72 h.

For the inventive microorganism, a temperature of about 30°C has been determined as the optimum temperature for PHA production. Therefore, the process of the present application is preferably run at temperatures of from about 15°C to 45°C and preferably from about 20°C to 40°C.

In an embodiment of the present application, which is different to the above-mentioned batch-process, the carbon source is supplied to the cultivating medium in a fed-batch manner, i.e. a manner, which involves the supplementation of an exponentially increasing carbon dosage after an initialization time of the fermentation. The parameters from the calculation of the exponentially increasing carbon dosage was calculated based on the following equation:

$$F(t) = \mu \cdot \frac{V_0 \cdot X_0}{S_0 \cdot Yx/s} \cdot e^{-\mu set \cdot t}$$

wherein F(t) is the flow rate of the carbon source along the cultivation, $V_0$ is the volume of the culture, $Y_{x/s}$ is the yield of biomass, $X_0$ is the initial biomass after the batch culture, $\mu_{set}$ is the desired specific growth rate, and So is substrate concentration in the feed.

$\mu_{set}$ in the inventive process is preferably in the range of about 0.05 to 0.1 $h^{-1}$, more preferably in the range of about 0.06 to 0.085 $h^{-1}$.

The above-mentioned fed-batch process allows for a substantial reduction of the fermentation time to reach maximum yield, wherein the optimum PHA concentration in the fermentation could be reduced to a range of about 40 to 48 h. This represents significant advantages over the conventional batch process, wherein an optimum PHA concentration is usually obtained only after about 72 h.

In the afore-mentioned process, it is preferred that prior to the addition of an exponentially increasing carbon source dosage, the fermentation is initialized in a batch phase wherein an initial lump of carbon source is added to the cultivating medium and the culture is subsequently maintained for a time sufficient to ensure complete initial carbon source consumption. In the practice of the present invention it has been observed that the initial batch phase is suitably carried out for a time of from about 12 to 22 h. Preferably the initial phase of the fed-batch process is carried out for about 12 to 15 h.

In the fed-batch process, it is further preferred that the initial lump of carbon source provides a carbon source concentration in the cultivating medium in the range of about 10 to 20 mM, preferably from about 12 to 17 mM. This range had been determined to provide optimal initial cultivation before onset of the exponential feeding process.

The stirring rate of the fermentation mixture in the batch or fed batch process is not subject to any relevant limitations except that it has to be sufficient to maintain an oxygen pressure in the above-indicated ranges. Suitable stirring rates depend on the requirements of the fermentation, but are usually within the range of about 200 to 1400 rpm.

The microorganism of the present invention has unexpectedly been discovered to exhibit fusion of PHA granules to a single granule during the fermentation, while initially multiple PHA granules were formed.

As concerns the isolation of the PHA from the microorganisms, it is preferred that a PHA is extracted with a non-chlorinated solvent, preferably with a ketone having 3 to 8 carbon atoms. Non-chlorinated solvents provide the advantage of significantly lower waste disposal problems and costs compared to conventional chlorinated solvents such as chloroform and dichloromethane. The referred ketones for use in the practice of the present application are acetone, 2-methylethylketone, diethylketone, 2-methylpropylketone, etc. The most preferred ketone for use in the isolation of PHA is acetone.

It is further preferred that the PHA is extracted at temperatures of less than about 60°C, preferably at temperatures of from about 20°C to 40°C. It has unexpectedly been discovered that the extraction of the inventive microorganism at these temperatures provide substantially the same PHA yields as comparable extractions at higher temperatures. It is believed that this is a direct result from the formation of a single PHA-granule at high carbon concentrations and the observable disruption of microorganism's cell walls towards the end of the fermentation process. Thus, in the inventive microorganism, the PHA is easier to access for the solvents than the multiple granules in a microorganism of a conventional fermentation. It had further been observed that substantially the same yield of extracted PHA could be obtained after extractions for about 0.5 to 5 h. It is preferred that the solvent extraction is carried for a time of about 1 to 3 hours, preferably for about 1 hour.

A further aspect of the present application is PHA obtainable by the process as described above. Preferably, the process involves the incorporation of carboxylic acids comprising aromatic moieties and/or unsaturated moieties. More preferably, the PHA obtained by the process contains 5 to 20%-mol saturated, 30 to 70%-mol unsaturated and 20 to 60%-mol aromatic monomers.

A yet further aspect of the present application is the use of a microorganism as described above in a process for the production of medium- or long-chain PHAs. Preferred embodiments of this process are identical to those described for the process for the production of medium- or long-chain PHAs above.

A final aspect of the present application is the use a PHA synthase as deposited in the Gene Bank (NCBI) under the Accession number JN651419 (phaC1) or JN216884 (phaC2) or analogues thereof for the production of PHA. The PHA synthases or analogues thereof may be used either alone or in mixtures thereof. An "analogue" as this term is used in the practice of the present invention is indented to mean a peptide or protein, which has at least about 80% sequence identity, preferably at least about 90% sequence identity, more preferably at least about 95% sequence identity, and most preferably at least about 98% sequence identity, and has comparable properties in that it is capable to synthesize PHA under appropriate conditions and accepts and incorporates unsaturated carboxylic acids and/or carboxylic acids comprising aromatic moieties into PHA. In a preferred embodiment, the use is for the production of PHA comprising one or more of unsaturated carbon-carbon double bonds and aromatic moieties, preferably phenyl moieties.

In the following, the present application will be described further by way of examples, which, however, are not intended to limit the scope of the present application by any means.

## Example 1

In order to select the best media for PHA production, *Pseudomonas sp.* IPB-A36 was cultured in three different media (MM+0.1%YE, R2A and C-Y) in 500 ml flasks (100 ml culture) at 30°C and 200 rpm and 10-undecenoate (27 mM) as the carbon source.

Table 1 Biomass and PHA production from *Pseudomonas sp.* IPB-A36 using different media

|  | MM+0.1%YE[1] | R2A[2] | C-Y[3] |
| --- | --- | --- | --- |
| **CDW (g/L)** | 0.65±0.10 | 1.41±0.06 | 1.69±0.15 |
| **PHA (g/L)** | 0.17±0.03 | 0.58±0.03 | 0.83±0.14 |
| **PHA (wt.-%)** | 25.7±3.9 | 41.0±1.9 | 48.8±3.6 |

Values were obtained after 72 h of incubation at
30°C and 200 rpm and are means of triplicates ±
standard deviation.
[1] Martinez-Blanco et al., 1990, J. Biol. Chem. 265: 7084-7090
[2] Reasoner & Geldreich, 1985, Appl. Environ. Microblol. 49: 1-7
[3] Choi et al., 1994, Appl. Environ. Microbiol. 60: 3245-54

The best results were obtained when medium C-Y was used, obtaining 1.69 g/L and 48.8 wt.-% of biomass and PHA accumulation, respectively.

## Example 2

In order to improve the biomass and PHA yield, the contents of nitrogen (N) and carbon (C) were modified. This experiment was carried out in 1 L flasks containing 200 ml culture, at 30°C and 200 rpm. Two different concentrations of nitrogen (N and 2N) and carbon source (27 mM and 54 mM) were assayed. The standard conditions employ concentrations of nitrogen and carbon source in C-Y medium of 0.66 g/L or 5 mM $(NH_4)_2SO_4$ (N) and 27 mM of carbon source. In 2N the $(NH_4)_2SO_4$ concentration was 1.32 g/L or 10 mM.

Table 2. *Pseudomonas sp.* IPB-A36 biomass and PHA production at different (C/N) ratio

| C11:1 (mM) | $(NH_4)_2SO_4$ (g/L) | Ratio (C/N) | CDW (g/L) | PHA (g/L) | PHA (wt.-%) |
| --- | --- | --- | --- | --- | --- |
| **27** | **0.66** | **30** | 2.10 | 0.82 | 39.0 |
| **54** | **0.66** | **60** | 1.26 | 0.47 | 37.3 |
| **27+27‡** | **0.66** | **~30** | 1.40 | 0.76 | 54.3 |
| **54** | **1.32** | **30** | **3.50** | **1.77** | **50.6** |
| **027+27‡** | **1.32** | **~15** | 2.86 | 1.23 | 43.0 |

**(27+27) ‡** indicates that the starting carbon source concentration was 27 mM and after 24 h
of culturing, a new pulse of 27 mM of carbon source was added. Values were obtained after
72 h of incubation at 30°C and 200 rpm and are means of duplicates.

As can be seen from Table 2, the best yields were obtained, using 54 mM of C11:1 and 1.32 g/L of $(NH_4)_2SO_4$, indicating that by increasing the concentration of carbon and nitrogen by two-fold, and maintaining the C/N ratio at 30, the PHA production showed a two-fold increase.

The samples obtained after fermentation with 27 mM C11:1, 27 + 27 mM C 11:1 and 54 mM C11:1 and 0.66 g/L $(NH_4)_2SO_2$ were investigated with a microscope. Figure 1 shows the effects of the carbon source on granule formation in an initial stage and after 72 h of cultivation. When the culture was supplied with 27 mM of substrate, several granules (Fig. 1-1A and 1B) were observed, whereas at higher carbon source concentrations (Fig. 1-2A and 2B, and Fig. 3-3A and 3B) most of the cells contained only unique large granules occupying the total cytoplasm space. The morphology observed suggests that the size of granules might contribute to the cell lysis.

The effect of the concentration of the nitrogen source on granule formation was also investigated. At 1.32 g/L $(NH_4)_2SO_4$ (2N), multiple large granules per cell were observed and bacterial cells appear to be healthier than the ones cultured in the normal medium C-Y in the initial fermentation stage. In general, a good PHA accumulation could be observed and the images are in good agreement with the quantitative results reported in Table 2. The changes in the granule formation process at 72 h of cultivation suggest that the size of the granules could positively influence the PHA recovery during the downstream processes.

## Example 3

*Pseudomonas sp.* IPB-A36 was cultured in 100 ml flasks containing 20 ml of C-Y medium at 30°C and 200 rpm using different substrates to investigate the influence of a co-substrate in the PHA structure. PHA production in 10-undecenoate was used as control, and two different aromatic substrates, [5-phenylvalerate (5-PheVal) and 8-phenyloctanoate (8-PheOct)] as well as combinations of unsaturated/aromatic substrates, were assayed (Table 3).

The aromatic substrates were tested first for their toxicity to the bacterial cells. Table 3 shows that the strain was able to grow and accumulate PHA when was cultivated either in 5-phenylvalerate or 8-phenyloctanoate as a unique carbon source. However, low PHA yields were obtained, being 15-18 wt.-% and 7 wt.-% for 5-phenylvalerate and 8-phenyloctanoate, respectively. PHA yields increased up to 40-50 wt.-%, when the aromatic substrate was co-fed with 10-undecenoate (14 or 27 mM).

**Table 3** *Pseudomonas sp.* IBP-A36 biomass and PHA production using aromatic substrates.

| substrate | CDW (g/L) | PHA (g/L) | PHA (wt.-%) |
|---|---|---|---|
| **C11:1 (14 mM)** | 0.97 | 0.4 | 36.2 |
| **C11:1 (27 mM)** | 2.45 | 1.2 | 49.7 |
| **5-PheVal (2 mM)** | 0.88 | 0.1 | 15.1 |
| **5-PheVal (5 mM)** | 0.65 | 0.1 | 17.9 |
| **5-PheVal (10 mM)** | 1.47 | 0.3 | 17.0 |
| **C11:1 (14 mM)+5-PheVal (2 mM)** | 2.17 | 0.9 | 43.1 |
| **C11:1 (14 mM)+5-PheVal (10 mM)** | 2.40 | 0.9 | 38.2 |
| **C11:1 (27 mM)+5-PheVal (2 mM)** | 2.00 | 0.7 | 36.7 |
| **C11:1 (27 mM)+5-PheVal (5 mM)** | 2.20 | 0.9 | 42.4 |
| **C11:1 (27 mM)+5-PheVal (10 mM)** | 2.45 | 1.2 | 49.7 |
| **8-PheOct (5 mM)** | 1.02 | 0.1 | 6.6 |
| **C11:1 (14 mM)+8-PheOct (5 mM)** | 2.03 | 0.9 | 42.6 |

Values were obtained after 72 h of incubation at 30°C and 200 rpm.
**5-Pheval:** 5-phenylvalerate
**8-PheOct:** 8-phenyloctanoate
**C11:1:** 10-undecenoate

It was observed that if 5-phenylvalerate was used in combination with 10-undecenoate (27 mM), *Pseudomonas sp.* IPB-A36 accumulated a PHA polymer that contained 2 to 5% aromatic monomers. Although this percentage was low, significant changes in the thermal properties of the obtained polymer were observed.

The PHA was investigated by NMR-spectroscopy and GC-MS, which provided the results shown in Table 4.

**Table 4** Monomer composition of the PHA polymers obtained when a mixture of 10-undecenoate and 5-phenyl-valerate is used a substrate

| Substrate | Unsaturated monomers (vinyl group), rel. %mol | | | Aromatic monomers (rel. %mol) | | | Others rel. %mol |
|---|---|---|---|---|---|---|---|
| | 3OHC7:1 | 3OHC9:1 | 3OHC11:1 | 5-Phe-3OHC5 | 6-Phe-3OHC6 | 8-Phe-3OHC8 | |
| C11:1 (14mM)+ 5-PheVal (2 mM) | 10.4 | 32.0 | 19.5 | 26.0 | | | 12.0 |
| C11:1 (14mM)+ 5-PheVal (5 mM) | 5.9 | 21.0 | 10.1 | 53.0 | | | 10.0 |
| C11:1 (14mM)+ 8-PheOct (5 mM) | 13.0 | 32.2 | 19.8 | | 13.3 | 9.7 | 12.0 |

**3OHC11:1:** 3-hydroxy-10-undecenoate
**3OHC9:1:** 3-hydroxy-8-nonenoate
**3OHC7:1:** 3-hydroxy-6-heptenoate
**5-Phe-3OHC5:** 5-phenyl-3-hydroxy-valerate
**8-Phe-3OHC8:** 8-phenyl-3-hydroxy-octanoate

The NMR-analysis indicates the presence of 10-12% of saturated monomers identified as 3-hydroxyoctanoate and 3-hydroxydecanoate. The presence of the saturated monomers might be a consequence of the strain using other metabolic pathways (e.g., *de novo* synthesis of fatty acids) besides the β-oxidation to synthesize polyhydroxyalkanoates. When 2 mM of the 5-phenylvalerate was supplied as a co-feeding, the relative %-mol of aromatic monomers amounted to 26%-mol, while the percentage increased to up to 53%-mol, when 5 mM of 5-phenylvalerate was supplied.

When 5 mM of 8-phenylvalerate was used as a co-substrate, the polymer composition shifted towards 23%-mol of aromatic monomers, 65%-mol of unsaturated monomers and 12%-mol of saturated (C8:0 and C10:0) monomers.

Further analytical data of the prepared PHA's is presented in the following Table 5.

**Table 5** Molecular weight distribution of the different polymers produced by strain IPB-A36

| | $M_n$ (kDa) | $M_w$ (kDa) | $M_p$ (kDa) | Dispersity (PDI) |
|---|---|---|---|---|
| *Pseudomonas sp.* **IPB-A36 CY C11-1 (27 mM)** | 308 | 662 | 601 | 2.2 |
| *Pseudomonas sp.* **IPB-A36 CY C11:1** (27 mM)+5PheVal(2 mM) | 201 | 440 | 334 | 2.2 |
| *Pseudomonas sp.* **IPB-A36 CY C11:1 (14 mM)+5PheVal(5 mM)** | 70 | 198 | 99 | 3.0 |
| *Pseudomonas sp.* **IPB-A36 CY C11:1 (14 mM)+8PheOct(5 mM)** | 236 | 429 | 376 | 1.8 |

Values were determined by GPC (universal calibration): $M_p$ is the molecular weight at peak maximum; $M_n$, molecular weight in number, $M_w$, molecular weight in mass and PDI is polydispersity index.

The polymers produced by *Pseudomonas sp.* IPB-A36 grown from different substrate combinations display similar molecular-weight distributions, except *Pseudomonas sp.* IPB-A36 C11:1 (14 mM) + 5 PheVal (5 mM) that has significant lower molecular weight and exhibits the highest PDI. The DSC analysis shown in Table 6 also suggests different behaviour of this polymer in comparison to the rest of the PHA polymers analyzed.

**Table 6** Thermal properties of the different polymers produced by strain IPB-A36

| | $T_{a,1}$ (°C) | $T_{a,2}$ (°C) | $\Delta c_{p,1}$ (J g$^{-1}$ K$^{-1}$) | $\Delta c_{p,2}$ (J g$^{-1}$ K$^{-1}$) | $T_{q/c}$ (°C) | $T_{d,1}$ (°C) | $\Delta H_{d,1}$ (J g$^{-1}$) |
|---|---|---|---|---|---|---|---|
| **IPB-A36 CY C11:1 (27mM)** | -51 | | 0.49 | | -58 | 299 | 550 |

(continued)

| | $T_{a,1}$ (°C) | $T_{a,2}$ (°C) | $\Delta c_{p,1}$ (J g$^{-1}$ K$^{-1}$) | $\Delta c_{p,2}$ (J g$^{-1}$ K$^{-1}$) | $T_{q/c}$ (°C) | $T_{d,1}$ (°C) | $\Delta H_{d,1}$ (J g$^{-1}$) |
|---|---|---|---|---|---|---|---|
| IPB-A36 C-Y C11:1 (27 mM)+5PheI(2 nm) | -49 | -18 | 0.15 | 0.19 | | 300 | 560 |
| IPB-A36 C-Y C11:1 (14 mM)+5PheVal(5 mM) | -52 | -1 | 0.11 | 0.23 | -5 | 301 | 620 |
| IPB-A36 C-Y C11:1 (14 mM)+8PheOct(5 mM) | -47 | -24 | 0.28 | 0.10 | | 301 | 510 |

$T_g$: glass transition temperature, $T_{g/c}$: cooling run temperature, $\Delta c_p$: change of heat capacity at $T_g$, $T_d$: melting temperature and $\Delta H_d$: melting enthalpy. All data obtained from DSC second heating or first cooling run.

## Example 4

*Pseudomonas sp.* IPB-A36 was cultivated in the media C-Y and C-Y (2N) using oleic acid (1%) as a substrate. The best yields of biomass CDW (4.5 g/L) and PHA (2.1 g/L) were obtained when C-Y (2N) was used, although similar rates of PHA accumulation (~50 wt.-%) were observed under both conditions.

According to GC-MS and NMR analysis, the resulting PHA polymer was constituted by: 8 mol-% 3OHC6:0. 44.2 mol-% 30HC8:0, 24.5 mol-% 3OHC10:0, 10.7 mol-% C3OHC12:0 and 12.6 mol-% 3OHC14:1 (3OHC = 3-hydroxycarboxylic acid, the first number of e.g. 14:1 indicates the total number of carbon atoms, the second number the number of double-bonds). The further properties of this polymer were indicated in the following Table 7.

**Table 7** Molecular weight distribution of the PHA-polymers produced by IPB-A36

| Strain/medium-substrate | $M_n$ (kDa) | $M_w$ (kDa) | $M_p$ (kDa) | Dispersity PDI |
|---|---|---|---|---|
| IPB-A36/ C-Y- oleic | 94 | 194 | 147 | 2.1 |

| | $T_{g,1}$ (°C) | $\Delta c_{p,1}$ (J g$^{-1}$ K$^{-1}$) | $T_{g/c}$ (°C) | $T_{d,1}$ (°C) | $\Delta H_{d,1}$ (J g$^{-1}$) |
|---|---|---|---|---|---|
| IPB-A36/ C-Y- oleic | -48 | 0.42 | -52 | 298 | 520 |

Values were determined by GPC (universal calibration): $M_p$ is the molecular weight at peak maximum; $M_n$, molecular weight in number, $M_w$, molecular weight in mass and PDI is polidispersity index; $T_g$: glass transition temperature, $T_{g/c}$: cooling run temperature, $\Delta c_p$: change of heat capacity at $T_g$, $T_d$: melting temperature and $\Delta H_d$: melting enthalpy. All obtained from DSC second heating or first cooling run.

## Example 5

*Pseudomonas sp.* IPB-A36 was cultivated in a fed-batch process in medium C-Y(2N), using starting stirring of 400 rpm, an air flow rate of 3 L/min and the partial pressure of oxygen (pO2) fixed at 30% (relative to total gas dissolved in the medium) and maintained using cascade control. The kinetic parameters were calculated and a $\mu_{set}$ of 0.075 h$^{-1}$ was chosen. Additionally, an external pump for the NH$_4$$^+$ feeding was added. According to the calculations, the initial batch was extended until 15 h to assure complete carbon-source consumption, and followed by 44 h of exponential feeding.

After the initial 15h of cultivation, the carbon source was completely consumed (as determined by HPLC analysis) and the exponential feeding was started. The culture reacted immediately and the demand of oxygen increased due to the higher metabolic activity. The stirring speed was increased up to its maximum of 900 rpm, and pure oxygen needed to be supplied. The percentage of pure oxygen mixed in the air flow had to be increased until the end of the process and reached values up to 28%-mol.

Biomass and PHA production data are summarized in Table 8. The data shows that after 40 h of cultivation the cells stopped growing, but continued accumulating, indicating a possible problem with the nitrogen consumption.

**Table 8** Biomass and PHA production and OD measurement for the fed-batch process BR-5.12.

| time (h) | CDW (g/L) | CDW-liof (g/L) | res biom (g/L) | PHA (g/L) | PHA (%wt) | OD$_{(550\,nm)}$ |
|---|---|---|---|---|---|---|
| 0 | 0.02 | 0.00 | 0.02 | 0.00 | 0.0 | 0.200 |
| 11 | 1.15 | 1.07 | 0.57 | 0.58 | 50.4 | 5.698 |
| 13 | 1.72 | 1.72 | 0.95 | 0.77 | 44.8 | 8.704 |
| 17 | 2.72 | 2.68 | 1.50 | 1.22 | 44.9 | 13.853 |
| 20 | 4.70 | 4.50 | 2.59 | 2.11 | 44.9 | 26.677 |
| 22 | 5.31 | 4.95 | 2.64 | 2.67 | 50.3 | 36.192 |
| 25 | 5.61 | 4.84 | 2.68 | 2.93 | 52.3 | 39.715 |
| 29 | 6.31 | 6.29 | 2.48 | 3.83 | 60.7 | 37.944 |
| 32.5 | 6.77 | 6.67 | 2.77 | 4.00 | 59.1 | 33.738 |
| 36.5 | 8.36 | 8.54 | 3.53 | 4.83 | 57.8 | 45.348 |
| 38.5 | 9.67 | 9.21 | 4.73 | 4.94 | 51.1 | 55.219 |
| 42 | 10.25 | 10.68 | 4.18 | 6.07 | 59.2 | 65.295 |
| 46 | 11.10 | 11.08 | 4.56 | 6.54 | 58.9 | 73.44 |
| 50 | 11.21 | 11.51 | 4.35 | 6.86 | 61.2 | 65.418 |
| 54 | 11.30 | 11.22 | 4.16 | 7.14 | 63.2 | 62.420 |
| 70 | 11.77 | 11.57 | 4.36 | 7.41 | 63.0 | 76.095 |

Values are means of duplicate

PHA (wt.-%) accumulation was higher at earlier stages of the fermentation, reaching values between 45-60 wt.-% along the whole process. It is remarkable that under these culture conditions the strain *Pseudomonas sp.* IPB-A36 was able to synthesize more PHA (7.41 g/L) than to grow, being the residual biomass (biomass free of PHA) about 4.5 g/L.

**Example 6**

*Pseudomonas sp.* IPB-A36 was cultivated in a further improved fed-batch process, following essentially the same conditions used in Example 5 with medium C-Y (2N), using starting stirring of 400 rpm, an air flow rate of to 3 L/min, and a pO2 fixed at 30%-mol using cascade control. The kinetic parameters were re-calculated and a $\mu_{set}$ of 0.075 h$^{-1}$ was fixed. The process started with a batch culture with 2.5 g/L of C11:1 during the initial 14 h of batch fermentation, followed by an exponential feeding over 45 h.

After the initial 14 h of cultivation, the carbon-_source was completely consumed (as detected by HPLC analysis) and the exponential feeding was started. The growth process in the batch culture finished earlier than expected, after 10 h of cultivation. However, as soon as the exponential feeding started, the culture reacted immediately as observed by the drastic increase of the required stirring and the oxygen consumption due to the higher metabolic activity. The stirring speed was increased up to 1,400 rpm and the gas flow needed a mixture of 60% of pure oxygen to keep the pO$_2$ at 30%.

The ammonium feeding was affecting directly the polymer accumulation. The PHA accumulation decreased considerably during the phase of maximal growth (between 24 h and 36 h of cultivation) as reflected in Figure 2A. The ammonium content in the media was kept at around 400 mg/L (about 22 mM) to ensure bacterial growth (Figure 2B). PHA accumulation started again after 36 h of cultivation, as indicated by the decrease in ammonium consumption.

At 42 h of cultivation, an increase of the foam formation was observed. The cultivation was stopped at 45 h. Isolation of the PHA produced by the microorganisms provided a cell dry weight of 22.5 g/L and a PHA yield of 8.9 g/L, respectively.

**Example 7**

The impact of PHA granule coalescence in the PHA recovery was evaluated by means of a solvent extraction method. PHA extraction has been conducted in two different solvents (acetone and chloroform), at different extraction temperatures (room temperature and 80°C) and different times of extraction (1 h and 3 h). Two different culture conditions were chosen, in order to evaluate the two different morphologies that were observed in the granule formation: (i) multiple granule formation distributed along the cytoplasm and (ii) formation of a unique big granule occupying the totality of the bacterial cell.

*Pseudomonas sp.* IPB-A36 was cultured at 30°C and 200 rpm in C-Y medium using two different carbon-_source concentrations: (a) 27 mM of C11:1 and (b) 27+27 mM, meaning that a pulse of 27 mM of C11:1 was added after 24 h of culturing. Cells were harvested after 72 h of cultivation and freeze dried to be later extracted, using the different extraction conditions described above. Samples of 40 mg of lyophilized biomass were disposed in the extraction tubes, re-suspended in the corresponding solvent and extracted. Percentages of PHA recovery are summarized in Table 9.

**Table 9** Percentage of PHA recovery obtained using different extractions conditions.

| Substrate | solvent | 1 h-RT | 3 h-RT | 1 h-80°C | 3 h-80°C |
|---|---|---|---|---|---|
| 27 mM C11:11 | chloroform | 44.2±1.8 | 44.9±1.0 | 48.1±0.2 | 47.3±1.1 |
| | acetone | 43.1±2.4 | 38.7±1.0 | | |
| 27+27 mM C11:11 | chloroform | 58.6±2.5 | 60.4±8.1 | 59.9±0.9 | 58.2±1.9 |
| | acetone | 55.1±1.6 | 54.8±0.6 | | |

Results are means of triplicates ± standard deviation. RT: room temperature

The highest percentage of PHA recovery was obtained, in both culture conditions, when chloroform was used as extractor solvent, being of 44-48% in the cultures with 27 mM of C11:1 and 58-60% in the case of the cultures with 27+27 mM of C11:1.

The classical extraction with chloroform (3 h and 80°C) was considered as the maximum percentage of PHA recovery (100%) and used as control to calculate a relative percentage of PHA recovery, in order to evaluate whether there was any difference among the two granules morphologies. Chloroform extraction at room temperature, independently of the extraction time, showed a slight difference (5% aprox.) among the two granule morphologies. The relative percentage of recovery was 95% in the case of the cultures with 27 mM C11:1 (multiple granules) and 100% in the case of the cultures with 27+27 mM C11:1 (unique big granule). Nevertheless, no differences were observed in the relative percentages (rel. %) of recovery when chloroform was used at 80°C. In the case of using acetone as solvent, the relative percentage of recovery was lower (85-95 rel.%) than the ones obtained with chloroform (95-100 rel.%) and slight differences (5-8 rel.%) were detected between the two morphologies. A 5-8 % increment in the relative percentage of recovery was found.

**Claims**

1. A microorganism of the genus *Pseudomonas* as deposited under DSM26198 with the DSMZ.

2. A process for the production of medium- or long-chain PHA comprising

   - cultivating a microorganism of the genus *Pseudomonas* as deposited under DSM26198 with the DSMZ in a culture medium comprising a carbon source and
   - isolating the PHA from the microorganism.

3. A process according to claim 2, wherein the medium is C-Y medium, preferably C-Y(2N) medium.

4. A process according to claim 2 or 3, wherein the carbon source comprises

   - at least one C4 to C20 fatty acid, preferably a C8 to C18 fatty acid, said fatty acid(s) optionally comprising one or more unsaturated moieties,
   - at least one carboxylic acid comprising an aromatic moiety, preferably an ω-phenyl substituted fatty acid, more preferably comprising 4 to 10 carbon atoms in the fatty acid chain, or mixtures thereof.

5. A process according to claim 4, wherein a mixture of saturated and/or unsaturated fatty acids and carboxylic acids comprising one or more unsaturated moieties is co-fed to the culture medium.

6. A process according to any one of claim 2 to 5, wherein the nitrogen is present in the culture medium as an ammonium salt, preferably with a molar ammonium concentration in the range of about 8 to 30 mM, in particular in the range of 10 to 20 mM.

7. A process according to any one of claim 2 to 6, wherein the process is a shake-flask- or batch-process and the carbon to nitrogen (C/N) ratio in the culture medium is in the range of about 20 to 45, preferably in the range of about 25 to 35.

8. A process according to any one of claim 2 to 6, wherein the carbon source is supplied to the cultivating medium in a fed-batch manner to provide an exponentially increasing carbon source dosage after an initial batch phase, preferably with a specific growth rate $\mu_{set}$ in the range of 0.05 to 0.1 $h^{-1}$, more preferably in the range of 0.06 to 0.085 $h^{-1}$.

9. A process according to claim 8, wherein in the batch phase an initial lump of carbon source is added to the cultivation medium and the culture is maintained for a time sufficient to assure complete consumption of the initial carbon-source, preferably, wherein the initial batch phase is maintained for 12 to 22h, more preferably wherein the initial batch phase is maintained for 12 to 15 h.

10. A process according to claim 8 or 9, wherein the initial lump of carbon source provides a carbon source concentration in the cultivating medium in the range of about 10 to 20 mM, preferably about 12 to 17 mM.

11. A process according to any one of claims 2 to 10, wherein the PHA is extracted with a ketone having 3 to 8 carbon atoms, preferably with acetone.

12. A process according to claim 2 to 11, wherein the PHA is extracted at a temperature of about 60°C or less, preferably at about 20 to 40°C.

13. PHA obtainable by the process of any one of claims 2 to 12, wherein the PHA preferably contains unsaturated and/or aromatic moieties, more preferably with relative mol% ratios of 5 to 20% saturated, 30 to 70% unsaturated and 20 to 60% aromatic monomers.

14. Use of a microorganism according to claim 1 in a process for the production of medium- or long-chain PHA.

15. Use of a PHA synthase as deposited in the Gene Bank (NCBI) under the Accession number JN651419 (*phaC1*) or JN216884 (*phaC2*) or analogues thereof or mixtures of these PHA synthases or analogues thereof for the production of PHA, preferably PHA containing carbon-carbon double bonds and/or aromatic moieties.

Figure 1

**Figure 2**

## EUROPEAN SEARCH REPORT

Application Number

EP 13 17 3575

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FURRER ET AL: "Efficient recovery of low endotoxin medium-chain-length poly([R]-3-hydroxyalkanoate) from bacterial biomass", JOURNAL OF MICROBIOLOGICAL METHODS, ELSEVIER, AMSTERDAM, NL, vol. 69, no. 1, 28 March 2007 (2007-03-28) , pages 206-213, XP005928196, ISSN: 0167-7012, DOI: 10.1016/J.MIMET.2007.01.002 * abstract * | 1-14 | INV. C12R1/38 C12N9/10 C12P7/62 |
| X | ALEXANDER MUHR ET AL: "Novel Description of mcl-PHA Biosynthesis by Pseudomonas chlororaphis from Animal-Derived Waste", JOURNAL OF BIOTECHNOLOGY, vol. 165, no. 1, 1 May 2013 (2013-05-01), pages 45-51, XP55084933, ISSN: 0168-1656, DOI: 10.1016/j.jbiotec.2013.02.003 * abstract * | 1-14 | |
| X | Rosa Palmeri ET AL: "Polyhydroxyalkanoates (PHAs) Production Through Conversion of Glycerol by Selected Strains of Pseudomonas Mediterranea and Pseudomonas Corrugata", chemical engineering transactions, 31 December 2012 (2012-12-31), pages 121-126, XP55084937, Retrieved from the Internet: URL:http://www.aidic.it/cet/12/27/021.pdf [retrieved on 2013-10-22] * abstract * | 1-14 | TECHNICAL FIELDS SEARCHED (IPC) C12R C12N C12P |

-/--

The present search report has been drawn up for all claims

| Place of search | Date of completion of the search | Examiner |
|---|---|---|
| The Hague | 23 October 2013 | van Voorst, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

| | Application Number |
|---|---|
| | EP 13 17 3575 |

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIN SONG ET AL: "Polyhydroxyalkanoate (PHA) production using waste vegetable oil by Pseudomonas sp. strain DR2.", JOURNAL OF MICROBIOLOGY AND BIOTECHNOLOGY, vol. 18, no. 8, 1 August 2008 (2008-08-01), pages 1408-1415, XP55084938, ISSN: 1017-7825 * abstract * | 1-14 | |
| X | SIMON-COLIN C ET AL: "A novel mcl PHA-producing bacterium, Pseudomonas guezennei sp nov, isolated from a 'kopara' mat located in Rangiroa, an atoll of French Polynesia", JOURNAL OF APPLIED MICROBIOLOGY, OXFORD, GB, vol. 104, no. 2, 1 February 2008 (2008-02-01), pages 581-586, XP002472186, ISSN: 1364-5072, DOI: 10.1111/J.1365-2672.2007.03667.X * abstract * | 1-14 | |
| X | TAEK HO YANG ET AL: "Tailor-made type II Pseudomonas PHA synthases and their use for the biosynthesis of polylactic acid and its copolymer in recombinant Escherichia coli", APPLIED MICROBIOLOGY AND BIOTECHNOLOGY, vol. 90, no. 2, 1 April 2011 (2011-04-01), pages 603-614, XP55051491, ISSN: 0175-7598, DOI: 10.1007/s00253-010-3077-2 * abstract * | 13,15 | TECHNICAL FIELDS SEARCHED (IPC) |

-/--

The present search report has been drawn up for all claims

| Place of search | Date of completion of the search | Examiner |
|---|---|---|
| The Hague | 23 October 2013 | van Voorst, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**
**European Patent Office**
**Office européen des brevets**

# EUROPEAN SEARCH REPORT

Application Number

EP 13 17 3575

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIAO-WEN SHEN ET AL: "Engineering of polyhydroxyalkanoate (PHA) synthase PhaC2ofvia site-specific mutation for efficient production of PHA copolymers", APPLIED MICROBIOLOGY AND BIOTECHNOLOGY, SPRINGER, BERLIN, DE, vol. 91, no. 3, 21 April 2011 (2011-04-21), pages 655-665, XP019927349, ISSN: 1432-0614, DOI: 10.1007/S00253-011-3274-7 * abstract * | 13,15 | |
| X | MANFRED SCHMID ET AL: "Autoxidation of Medium Chain Length Polyhydroxyalkanoate", BIOMACROMOLECULES, vol. 8, no. 2, 1 February 2007 (2007-02-01), pages 579-584, XP55084983, ISSN: 1525-7797, DOI: 10.1021/bm060785m * abstract * | 13 | |
| X | DATABASE UniProt [Online] 18 April 2012 (2012-04-18), "SubName: Full=Class II poly(3-hydroxyalkanoic acid) synthase;", XP002715233, retrieved from EBI accession no. UNIPROT:H6UJH7 Database accession no. H6UJH7 * the whole document * | 13,15 | **TECHNICAL FIELDS SEARCHED (IPC)** |

-/--

The present search report has been drawn up for all claims

| Place of search | Date of completion of the search | Examiner |
|---|---|---|
| The Hague | 23 October 2013 | van Voorst, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 13 17 3575

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>6 February 2013 (2013-02-06),<br>"SubName: Full=Poly(3-hydroxyalkanoic acid) synthase class II;",<br>XP002715234,<br>retrieved from EBI accession no.<br>UNIPROT:K7PFD9<br>Database accession no. K7PFD9<br>* the whole document * | 13,15 | |
| X | WARD P G ET AL: "Bacterial synthesis of polyhydroxyalkanoates containing aromatic and aliphatic monomers by Pseudomonas putida CA-3",<br>INTERNATIONAL JOURNAL OF BIOLOGICAL MACROMOLECULES, ELSEVIER BV, NL,<br>vol. 35, no. 3-4,<br>1 April 2005 (2005-04-01), pages 127-133,<br>XP027766723,<br>ISSN: 0141-8130<br>[retrieved on 2005-04-01]<br>* abstract; table 2 * | 1-14 | TECHNICAL FIELDS SEARCHED (IPC) |

The present search report has been drawn up for all claims

| Place of search | Date of completion of the search | Examiner |
|---|---|---|
| The Hague | 23 October 2013 | van Voorst, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................................

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4876331 A
- US 5292860 A
- EP 1913135 A1

### Non-patent literature cited in the description

- **WILLIAMS ; PEOPLES.** *Chemtech.,* 1996, vol. 26, 38-44
- **LEMOIGNE ; ROUKHELMAN.** *Ann. Des Fermentation,* 1925, 527-536
- **DESMET et al.** *J. Bacteriol.,* 1983, vol. 154, 870-878
- **WALLEN ; ROHWEDER.** *Environ. Sci. Technol,* 1974, vol. 8, 576-579
- **STEINBÜCHEL ; WIESE.** *Appl. Microbiol. Biotechnol.,* 1992, vol. 37, 691-697
- **VALENTIN et al.** *Appl. Microbiol. Biotechnol,* 1992, vol. 36, 507-514
- **VALENTIN et al.** *Appl. Microbiol. Biotechnol.,* 1994, vol. 40, 710-716
- **ABE et al.** *Int. J. Biol. Macromol,* 1994, vol. 16, 115-119
- **LEE et al.** *Appl. Microbiol. Biotechnol,* 1995, vol. 42, 901-909
- **KATO et al.** *Appl. Microbiol. Biotechnol.,* 1996, vol. 45, 363-370
- **VALENTIN et al.** *Appl. Microbiol. Biotechnol,* 1996, vol. 46, 261-267
- **BRANDL et al.** *Int. J. Biol. Macromol.,* 1989, vol. 11, 49-55
- **AMOS ; MCINEREY.** *Arch. Microbiol,* 1991, vol. 155, 103-106
- **HRABAK.** *FEMS Microbiol. Rev.,* 1992, vol. 103, 251-256
- **CHOI ; LEE.** *Bioprocess Eng,* 1997, vol. 17, 335-342
- **STEINBUCHEL.** *Biomaterials,* 1991, 123-213
- **LEE.** *Biotech. Bioeng.,* 1996, vol. 49, 1-14
- **QI et al.** *FEMS Microbiol. Lett.,* 2007, vol. 157, 155-162
- **CAI et al.** *Bioresource Techn.,* 2009, vol. 100, 2265-2270
- **MARTINEZ-BLANKO et al.** *J. Biol. Chem.,* 1990, vol. 265, 7084-7090
- **REASONER ; GELDREICH.** *Appl. Environ. Microbiol.,* 1985, vol. 49, 1-7
- **CHOI et al.** *Appl. Environ. Microbiol,* 1994, vol. 60, 1245-1254
- **MARTINEZ-BLANCO et al.** *J. Biol. Chem.,* 1990, vol. 265, 7084-7090
- **REASONER ; GELDREICH.** *Appl. Environ. Microbiol.,* 1985, vol. 49, 1-7
- **CHOI et al.** *Appl. Environ. Microbiol.,* 1994, vol. 60, 3245-54